# EUROPEAN PATENT SPECIFICATION

(11) **EP 0 463 653 B1**
(45) Date of publication and mention of the grant of the patent: **25.01.1995**
(21) Application number: 91201211.9
(22) Date of filing: 16.05.1991
(51) Int. Cl.: A61K 47/48, A61K 9/06

(54) **Galenic formulations containing cyclodextrins**
Zyklodextrine enthaltende galenische Zusammensetzungen
Formulations galéniques contenant des cyclodextrines

(30) Priority: 07.06.1990 GB 9012663
(43) Date of publication of application: 02.01.1992
(73) Proprietor: PHARMACIA S.p.A., 20152 Milano (IT)
(72) Inventor: De Ponti, Roberto, I-20147 Milan (IT); Torricelli, Carla, I-20149 Milan (IT); Lardini, Ercole, I-20090 Pieve Emanuele (Milan) (IT); Martini, Alessandro, I-20145 Milan (IT); Mongelli, Nicola, I-20137 Milan (IT)
(74) Representative: Ferrario, Vittorino

(56) References cited:
- EP-A- 0 272 097
- EP-A- 0 300 526
- EP-A- 0 302 772
- EP-A- 0 308 181
- EP-A- 0 349 091
- WO-A-88/09163
- Hersey et al 1987 J.Pharm.Sci. 76:876-879
- Merkus et al 1993 J. Contro. Rel. 24.201-208
- De Ponti et al Minutes of the 6th Inter.Symp. on cyclodextrins 1992,Eds. de Sautè Paris pages 514-521

## Description

The present invention relates to new pharmaceutical compositions comprising cyclodextrins which are suitable for use in the delivery of drugs by absorption through mucosae. It also relates to a process for the preparation of such compositions and to the use of cyclodextrins in the manufacture of such preparations.

The administration of drugs by absorption through mucosae such as the nasal mucosa has been the subject of considerable interest in recent years. It has been proposed as an alternative to oral administration in cases where drugs are only absorbed poorly by an oral route or are extensively metabolised in the gastrointestinal tract or subjected to first-pass metabolism in the liver. Mucosal absorption is particularly attractive for products such as new physiologically acceptable proteins and polypeptides produced by the methods of biotechnology.

In particular the nasal mucosa has the desirable properties of low enzymatic activity and the avoidance of first-pass metabolism in the liver as the venous system from the nose passes directly into the circulatory system. The nasal mucosa also exhibits moderate permeability to water soluble compounds which is comparable to that of the ileum and gall bladder. This permeability is higher than that for other mucosae such as the vaginal mucosa: while the vaginal epithelium is composed of stratified squamous cells the nasal epithelium is comprised of columnar cells leading to the higher permeability. A high mucosal permeability is clearly desirable for the efficient absorption of drugs across the mucosa.

The efficient delivery of drugs by an intra-nasal route is influenced by the nasal clearance rate as well as the mucosa's permeability. This clearance rate is produced by the coordinated movement of cilia and is known to be highly dependent upon the prevailing physiological and pathological conditions. For instance it is known that both the common cold and polypectomy dramatically increase the nasal clearance rate which leads to a much decreased efficiency of uptake of drug by absorption through the nasal mucosa.

Accordingly, it has been, found that for many drugs administration by an intra-nasal route is inefficient due to low uptake of the drug and hence low bio-availability. Much effort has been applied to the development of methods for increasing the efficiency of absorption of drugs across mucosae such as the nasal mucosa. The most popular approach has been to increase the permeability of the mucosae and a large range of potential absorption enhancers which increase the permeability of mucosae have been developed. These are also sometimes called absorption or penetration promoters or enhancers.

Many types of compound have been proposed as absorption enhancers and these include chelating agents, fatty acids, bile acid salts, surfactants other than bile acid salts, fusidic acid and its derivatives, lysophasphatides and cyclic peptide antibiotics. However, many of these enhancers exhibit undesirable toxic side effects.

For example, chelating agents are known to interfere with the ability of calcium ions to control the paracellular transport of peptides and proteins [Lee, "delivery systems for peptide drugs", Plenum, New York (1986)] and are believed to play a vital role in the regulation of ciliary activity [Batts, J.Pharm. Pharmacol., 41, 156-159, (1989)]. Thus the chelating agent sodium edetate which is used in nasal preparations has been found to lead to irreversible damage of the ciliated epithelium at a concentration of 0.1% [Batts, 1989], and must be used a concentration lower than this, typically 0.05% [Hermans et al Pharmaceutical Research, 4(6), 445-449 (1987)].

Bile acid salts which can be used as absorption enhancers may be divided into two sub-classes, conjugated and unconjugated bile salts These include dihydroxy and trihydroxy bile acid salts such as 3α,12α-dihydroxy-5β cholanoates and 3α,7α,12α-trihydroxy-5β cholanoates. The more efficient absorption enhancers were more hydrophobic, for instance, sodium deoxycholate [Gordon et al, Proc. Natl. Acad. Sci. USA, 82, 7419-7423 (1985)]. It has been found that use of this compound as an absorption enhancer leads to an increase in the permeability of the nasal mucosa which is bidirectional and it is not reversed by washing. Histological evidence suggests that the increased permeability is due to damage caused by extensive loss of the epithelial surface layer. This makes bile acid salts unsuitable for use as absorption enhancers in patients [Hersey & Jackson, J.Pharm. M. Sci., 76, (12), 876-879, (1987)].

Other surfactants which have been used as nasal enhancers include saponin (a glycoside), surfactin (a peptide lipid), and Laureth-9 (a non-ionic detergent, polyoxyethylene-9 lauryl ether) [Hirai, Int. J.Pharm., 9, 165-172, (1981) and Duchateau et al, Int. J.Pharm., 39, 87-89, (1987)]. Laureth-9 shows one of the highest erythrocyte haemolysis effects as well as one of the higher effects of protein release from the nasal mucosa amongst known absorption enhancers [Hirai. Int. J.Pharm., 9, 173-184 (1981) and Longenecker et al, J.Pharm. Sei., 76, 351-355, (1987)]. A 1% solution of Laureth-9 has a "destructing action on the mucosal lining which included multi-focal necrosis, inflammation and exudation, and in some regions, complete removal of epithelial monolayer" [Daugherty et al, Int. J.Pharm., 45, 197-206, (1988)].

Thus the most important disadvantage with the use of many nasal enhancers is the possibility of side effects which after chronic administration cause irreversible damage to the nasal mucosa. While the absorption enhancer Na tauro 24,25-dihydrofusidate (STDHF) has been found to have good toxicological properties [Lee et al, BioPharm, April, 30-37, (1987)], there remains a need to reduce the toxicity of other absorption enhancers.

It has now been found that when an absorption enhancer is used in combination with a cyclodextrin the undesirable side effects due to the enhancer may be reduced. In particular it has been found that when a cyclodextrin is present the disruptive effects of absorption enhancers on lipid membranes may be reduced.

The present invention therefore provides pharmaceutical compositions suitable for administering a drug to a mucosal surface of a patient, which compositions comprise a drug, an absorption enhancer and a cyclodextrin, excluding
a) compositions wherein the absorption enhancer is at least one of succinic acid, tartaric acid, citric acid, fumaric acid, maleic acid, malonic acid, glutaric acid, adipic acid, malic acid, L-glutamic acid, L-aspartic acid, gluconic acid and glucuronic acid and wherein the drug is selected from the group consisting of calcitonin gene related peptides (CGRP), calcitonin, parathyroid hormone (PTH), insulin, somatostatin, growth hormone, secretin, gastrin, vaspressin, oxytocin, glucagon, adrenocorticotropic hormone (ACTH), thyroid-stimulating hormone (TSH), prolactin, luteinizing hormone-releasing hormone (LH-RH), endorphin, enkephalin, neurotensin, interferon, interleukin, superoxide dismutase and derivatives and salts thereof;
b) compositions wherein the absorption enhancer is didecanoyl phosphatidylcholine and wherein the drug the drug is selected from the group consisting of insulins, proinsulins, glucagon, parathyroid hormone, parathyroid hormone antagonist, calcitonin, vasopressin, renin, prolactin, growth hormone, thyroid stimulating hormone, corticotropin, corticotropin-releasing factor, follicle stimulating hormone, luteinizing hormone, chorionic gonadotropin, atrial peptides, interferon, tissue, plasminogen activator, gammaglobulins, Factor VII, Factor VIII, growth hormone releasing hormone, luteinizing hormone releasing hormone, somatostatin and cholecystokinins;
c) composition wherein the drug is 17β-oestradiol, progesterone or a combination thereof and wherein the cyclodextrin is dimethyl-β-ciclodextrin.
In particular the present invention provides compositions comprising a nasal absorption enhancer which are suitable for use as medicaments for therapy by an intra-nasal route.

The invention therefore also provides compositions for use in nasal administration of a drug to a mucosal surface and in particular the nasal mucosa. It also provides for the use of a cyclodextrin in the manufacture of a medicament suitable for administering a drug to a mucosal surface of a patient, the said medicament comprising a drug, an absorption enhancer and a cyclodextrin for reducing the toxic effects caused by the absorption enhancer.

As a further aspect of the invention there is provided a process for preparing a composition of the invention which comprised mixing a drug, an enhancer of absorption at a mucosal surface and a cyclodextrin.

Cyclodextrins are well known compounds formed by the breakdown of starch. For example, β-cyclodextrin is a cyclic oligosaccharide composed of 7 D-glucose units. Its structure involves a cylindrical cavity capable of including various guest molecules to form host-guest inclusion compounds in the solid state and in solution. Cyclodextrin inclusion compounds have been widely studied and developed for several fully soluble drugs.

The potential toxicity of cyclodextrins in relation to intra-nasal administration has been investigated. The effects of 2-hydroxypropyl β-cyclodextrin on human nasal ciliary epithelial function have been investigated [Messens, 1989 and Hermens et al, 47th Int. Congress of Pharm. Sciences of FIP Amsterdam 31.08-04.09 (1987)]. These studies concluded that this cyclodextrin exerts only a small effect on nasal ciliary movement. Chronic nasal administration in human volunteers has confirmed this excellent tolerance. No sign of irritation was observed after chronic application of the cyclodextrin on the eye in human volunteers or patients.

The cyclodextrin used in the present invention may be an α, β or τ cyclodextrin, for example depending on which cyclodextrin the enhancer in question binds to most efficiently. It is preferably a β-cyclodextrin because of the low cost of this compound and preferably it is a dehydrated cyclodextrin. β-dehydrated cyclodextrin is therefore especially preferred.

The term "dehydrated cyclodextrin", as used in the present invention, means a cyclodextrin, particularly β-cyclodextrin, which has a water content lower than 5%, preferably lower than 2%, by weight. Clearly, the above definition includes also a cyclodextrin which has the 0% of water content, that is an anhydrous cyclodextrin, for instance anhydrous β-cyclodextrin. Anhydrous β-cyclodextrin is a white crystalline powder, structurally different from hydrated β-cyclodextrin.

To obtain a dehydrated cyclodextrin e.g. dehydrated β-cyclodextrin, the commercially available cyclodextrin, that is a hydrated form containing, e.g. 12% to 14% by weight of water, is typically heated in an oven at a temperature which, depending on the heating time, may vary from about 100°C to about 220°C, preferably from about 100°C to about 140°C. Particularly preferred conditions involve the use of mechanical vacuum and a temperature ranging from about 115°C to about 130°C for 8 hours.

Alternatively, derivatives of α, β and τ cyclodextrins may be used where it is desirable to modify the characteristics of the parent cyclodextrins. In particular more soluble derivatives such as hydroxypropyl, di-methyl and tri-methyl substituted cyclodextrins, cyclodextrins linked with sugar molecules, sulfonated cyclodextrins, carboxylated cyclodextrins, and amino derivatives of cyclodextrins such as diethylaminoethyl cyclodextrins are preferred. A particularly preferred derivative is 2-hydroxypropyl-cyclodextrin. An alternative preferred derivative is a swellable polymer of a cyclodextrin. These differ in their swelling properties from the parent compounds. It is also preferred to use cyclodextrin derivatives which differ with respect of their toxicity from the parent compounds. For example, derivatives which caused reduced haemolysis or kidney damage would be preferred.

The enhancers to be used in combination with a cyclodextrin include chelating agents such as EDTA, EGTA, citric acid, salicylates, alginates, N-acylderivatives of collagen and enamines (N-amino acyl derivatives of β-diketones), fatty acids such as C8 and C10 chain length mono- and di-glyceride extracts of coconut oil (Capmul 8210 and Capmul MCM 90), monoolein or unsaturated fatty acids, bile acid salts such as 3α, 12α-dihydroxy-5β-cholanoates, Na deoxycholate, Na glycodeoxycholate, Na taurodeoxycholate, 3α, 7α, 12α-trihydroxy-5β-cholanoates_{,} Na cholate, Na glycocholate, Na taurocholate, other surfactants such as saponin (a glycoside), surfactin (a peptidelipid), a non-ionic detergent such as Laureth-9 (polyoryethylene-9-lauryl ether) and zwitterionic detergents such as (3-[3-cholamidopropyl-dimethylammonio 1-propane sulfonate), fusidic acid and its derivatives such as Na tauro 24,25-dihydrofusidate (STDHF) and Na glyco 24,25-dihydrofusidate (SGDHF) lysophosphatides such as lysophosphatidylcholine and in particular L-α-lysophosphatidylcholine, cyclic peptide antibiotics such as bacitracin and bacitracin A, preservatives such as methyl p-hydroxybenzoate, propyl p-hydroxybenzoate, chlorobutol, chlorocresol, benzalkonium chloride, chlorexidine and organo-mercuric compounds. Others suitable enhancers which may be used in combination with cyclodextrins include ascorbic acid, basic amino acids and/or their salts, glycyrrhetinic acid and glycyrrhizin, O-acylcarnitine derivatives like octanoyl carnitine, lauroyl carnitine, phosphatidyl carnitine and derivatives, palmitoyl carnitine, and malonates, e.g. diethyleneoxymethylene malonate.

Any active drug substance may be used in the composition of the present invention.

Examples of drugs are:
Proteins and peptides such as insulin (hexameric/dimeric/monomeric forms), gentamicin, glucagon, growth hormone (Somatotrophin), calcitonins and synthetic modifications thereof, enkephalins, interferons (especially Alpha-2 interferon for treatment of common colds), luteinising hormone-releasing hormone (LHRH) and analogues (Nafarelin, Buserelin, Leuprorelin, Goserelin), GHRH (growth hormone releasing hormone), secretin, nifedipine, bradykin antagonists, GRF (growth releasing factor), THF, TRH (thyrotropin releasing hormone), ACTH analogues, IGF (insulin-like growth factors) CGRP (calcitonin gene related peptide), atrial natriuretic peptide, vasopressin and analogues (DDAVP, lypressin), metoclopramide, migraine treatment (dihydroergotamine, ergometrine, ergotamine, pizotizin), nasal vaccines (particularly AIDS vaccines), FACTOR VIII;
Antibiotics and antimicrobial agents such as tetracycline hydrochloride, leucomycin, penicillin, penicillin derivatives and erythromycin, chemotherapeutic agents such as sulphathiazole and nitrofurazone; local anaesthetics such as benzocaine; vasoconstrictors such as phenylephrine hydrochloride, tetrahydrozoline hydrochloride, naphazoline nitrate, oxymetazoline hydrochoride and tramazoline hydrochloride; cardiotonics such as digitalis and digoxin; vasodilators such as nitroglycerin and papaverine hydrochloride; antiseptics such as chlorhexidine hydrochloride, hexylresorcinol, degualinium chloride and ethacridine; enzymes such as lysozyme chloride, dextranase; bone metabolism controlling agents such as vitamine D₃ and active vitamine D₃; sex hormones; hypotensives; sedatives; anxiolytics and anti-tumor agents;
Steroidal anti-inflammatory agents such as hydrocortisone, prednisone, fluticasone, predonisolone, triamcinolone, triamcinolone acetonide, dexamethasone, betamethasone, beclomethasone, and beclomethasone diproprioate; non-steroidal anti-inflammatory agents such as acetaminophen, aspirin, aminopyrine, phenylbutazone, mefenamic acid, ibuprofen, ibufenac, alclofenac, diclofenac sodium, indomethacin, colchicine, probenocid, phenactin, sulpyrine, sulfenamid acid; enzymatic anti-inflammatory agents such as chymotrypsin and bromelain seratiopeptidase; anti-histaminic agents such as diphenhydramine hydrochloride, chloropheniramine maleate and clemastine; anti-allergic agents (antitussive-expectorant antasthmatic agents such a sodium cromoglycate, codeine phosphate, and isoprotereol hydrochloride.

Other examples of active substances may be steroids, for instance medroxyprogesterone acetate (MPA), progesterone, testosterone, 6-methyleneandrosta-1, 4-diene-3,17-dione and the like; antibiotics such as e.g., griseofulvin, cefalosporin, penems and the like; antidepressant drugs such as, e.g., bensodiazepins, e.g. temazepam, oxazepam, diazepam, nitrazepam and the like; immunomodulators such as, e.g., 2-cyano-3-(1,4-dihydro-1-phenyl-(1)-benzothiopyran) (4,3-C)-pyrazol-3yl-3-oxo-N-phenylpropanamide and the like; antiinflammatory agents such as, e.g., indoprofen, ketoprofen, flufenamic acid and the like; antineoplastic agents such as, e.g. anthracycline glycosides, e.g. idarubicin (i.e. 4-dimethoxy daunorubicin) and 3'-desamine-3'-(3-cyano-4-morpholinyl)-doxorubicin and the like, etoposide, teniposide and other podophyllotoxins.

The compositions are especially suitable for the administration of physiologically active polypeptides.

All possible isomers, stereoisomers and optical isomers of drugs and their mixtures may be used in the compositions of the present invention. Metabolites and metabolic precursors or bioprecursors of drugs may also be used.

The relative proportions and dosages of the components in the compositions will vary with the nature of the components, the route of administration, the frequency of administration and the age, weight and condition of the patient. The compositions will comprise a sufficient amount of cyclodextrin to alleviate the damage to the mucosa caused by absorption enhancer.

The amount of drug, absorption enhancer and cyclodextrin may be decided on a case by case basis. The competition between a drug and an enhancer to form a complex with the cyclodextrin can be factor to consider. A drug and a cyclodextrin are suitably present in a composition according to the invention in a molar ratio of drug : cyclodextrin of from 1:0.05 to 1:30, for example from 1:0.5 to 1:3. Similarly, an enhancer and a cyclodextrin may be present in a molar ratio of enhancer: cyclodextrin of from 1:0.05 to 1:30, for example from 1:0.5 to 1:3.

The compositions may be suitable for administration by nasal, oral, rectal, buccal, intestinal, tracheal or vaginal routes, and especially nasal administration. They may further comprise additional ingredients as are conventionally found in such compositions, for instance suitable carriers, or diluents, as well as lubricants, binders, colourants, odor improvers, preservatives and surface-active agents.

Compositions of the present invention suitable for nasal administration may be in form of an aqueous solution for administration obtained by dissolving the drug, enhancer and cyclodextrin in the same aqueous medium.

Solid state preparations may also be successfully used for nasal administration. These may range from a simple physical mixture of the drug, enhancer and the cyclodextrin to a complex or inclusion compound between the enhancer and cyclodextrin mixed with the drug. Simple mixtures may be obtained for example by tumble mixing or sieving of the components and in this aspect of the invention the use of dehydrated cyclodextrin and in particular dehydrated β-cyclodextrin is preferred as a solution is formed on administration by water absorption from the mucosa. Inclusion complexes between the enhancer and cyclodextrin may be obtained by precipitation, freeze-drying, solvent evaporation, co-grinding, spray-drying, kneading or vapour solvent exposure. The preparation of such complexes is described for example in "Cyclodextrins and their industrial uses", Ed. Duchene, Editions de Sante, (1987) and Szejtli, Cyclodextrins and their inclusion complexes", Akademiai Kiado, Budapest, (1982). Inclusion complexes may be mixed with the drug by known methods, such as for example tumble mixing or sieving.

Solid compositions suitable for nasal delivery may for example be in the form of a powdery composition comprising at least about 90% by weight of a lower alkyl ether of cellulose as described in EP-A-28359. The cellulose ether is preferably methyl cellulose, hydroxy propyl cellulose or hydroxypropylmethyl cellulose and most preferably hydroxypropyl cellulose. Another suitable composition comprises a water-absorbing and water-insoluble base such as microcrystalline cellulose, as described in EP-A-0122036.

Alternatively, solid compositions suitable for nasal administration may comprise microspheres of starch derivatives, gelatin, albumin, collagen, dextran, dextran derivatives such as anionic derivatives for example a carboxylated or sulphated dextran or cationic derivatives for example diethylamino ethyl or a quaternary amino ethyl dextran, or similar materials as described in WO-A-88/09163.

The microspheres are typically water-swellable and water insoluble. Preferably they are starch or dextran derived and are crossed-linked microspheres composed of crossed-linked starch such as Spherex (Trade Mark). Such microspheres form a gel on administration which adheres to the mucosa and enhances absorbtion. This occurs through shrinkage of the epithelial cells, [Illum et al., Int.J. Pharm. 46, 261-265 (1988), Illum et al., Int. J. Pharm. 39, 189-199 (1987), Bjork et al., Int.J. Pharm., 47, 233-238 (1988) and Illum et al, 15th Int. Symposium on controlled delivery of bioactive material, August 15-19 (1988), Basel]. The microspheres are loaded with a drug, typically by freeze-drying a dispersion of microspheres in a solution of the drug. The microspheres may be loaded with or mixed with the absorption enhancers and/or cyclodextrin.

At least 90 wt % of particles of a composition for nasal delivery should have a particle diameter of from 10 to 250 »m such as from 10 to 100 »m and, more preferably, from 10 to 50 »m. Preferably all particles are within one of these size ranges. Solid state compositions may be administered intra-nasally by spraying. Such administration may be by use of a nasal insufflator device.

In vitro and in vivo experiments were carried out to test cyclodextrins' ability to protect the nasal mucosa from the undesirable effects of enhancers. The following experiments illustrate but don't limit the invention .

### In vitro experiments

It is well known that all animal cells are contained in a membrane whose constituents are mainly lipids, phospholipids, glycolipids, and proteins. This was simulated in a simple way using the phospholipid dipalmitoylphosphatidylcholine [DPPC] in such a condition to have a bilayer structure of the DPPC (the bilayer structure is encountered in cell membrane) [Yoshioka et al, J.Celloid Interface Science, 133(2), 442-446 (1989)].

It has now been found that the bilayer structure is destructured by Laureth-9 and that the addition of cyclodextrins has an effect in making this destructuring effect less prominent or negligible.

The addition of cyclodextrins is useful in making the parameters controlled in the in-vitro test reversible towards the initial values for DPPC alone. This experimental work, executed with an enhancer, Laureth-9, that is well known for destructuring membranes, demonstrates that cyclodextrins have a protective effect for the cell membranes making the interaction of the enhancer with the DPPC constituent of the in-vitro bilayer reversible.

A Differential Scanning Calorimetry (DSC) Mettler TA 3000 equipped with a TC10 processor, a DSC 20 cell (placed in a refrigerator) and a Wenger Matrix printer was used.

100 mg of DPPC (m.w. 734, Nippon Fine Chemical Lot. PA631215 or Sigma Lot. 116F-8365) were added to 1 mL of buffer solution, pH=7 (100 mL of the buffer solution at pH=7 were prepared adding 50 mL, of KH₂PO₄ 0.1M and 29 mL of NaOH 0.1M to a flask of 100 mL brought to volume with distilled water). The mixture was vigorously shaken and left for 1 hour at +50°C for annealing. 150»L of the mixture obtained were transferred into a 160»L aluminium pan and sealed immediately. As reference a 160»L sealed aluminium pan containing 150»L of buffer solution was used.

DSC runs were performed from +10°C to +80°C at a scan rate of 1K/min. The destructuring action of the absorbtion enhancers Laureth-9 (m.w. 582, Sigma Lot. 77-0458 [L9], L-α-Lysophosphatidylcholine Palmitoyl (m.w.496, Sigma Lot.70H-8383) [LPC], Deoxycholic Acid Sodium Salt (m.w.415, Sigma Lot.108F-0331) [DCH] was tested adding the weighted amount of enhancer to DPPC before adding 1ml of buffer solution. Blanks of all cyclodextrins tested were performed with no interference on the signals referred to DPPC; these were found in agreement with literature data [Yoshioka et al 1989 and Kelker H. and Hatz R., "Handbook of Liquid Crystals", Verlag Chemie, Basel; 1980, page 568, Figure 12.16].

The cyclodextrins used in this study were:
2-hydroxypropyl-β-cyclodextrin (m.w. about 1300, Roquette Lot. 0069 or Janssen Lot. 26322) [2-HP/βCD].
β-cyclodextrin tetradecasulfate (m.w. 2464, Farmitalia Carlo Erba R&D, Lot. CF/72 [βCDS],
α-cyclodextrin (m.w. 972, Wacker Chemie) [αCD],
τ-cyclodextrin (m.w. 1297, Wacker Chemie) [γ-CD]
Water soluble polymer of cyclodextrin (Chinoin Lot. CYL 97/1) [CYLCD].
Weighted amounts of the used cyclodextrins were added to DPPC or DPPC+L9 mixture before adding the buffer solution (1mL).

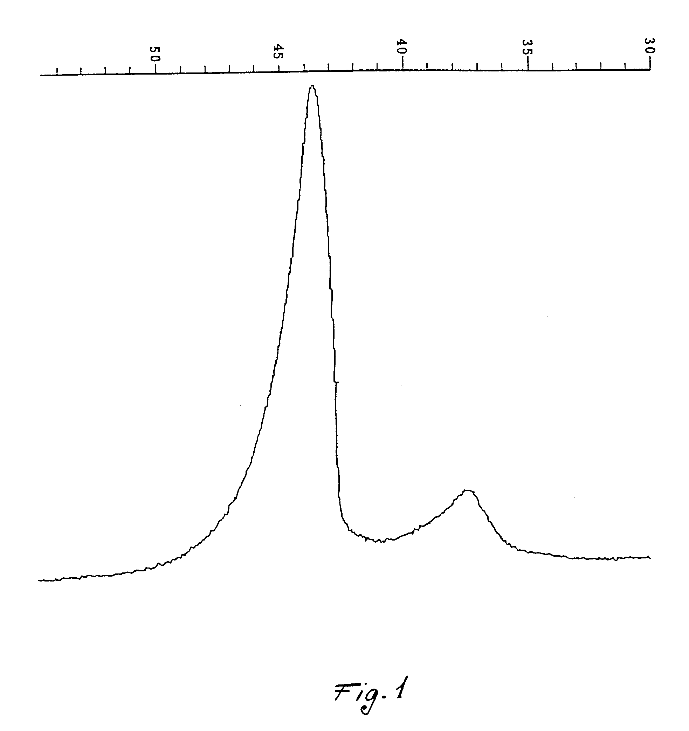

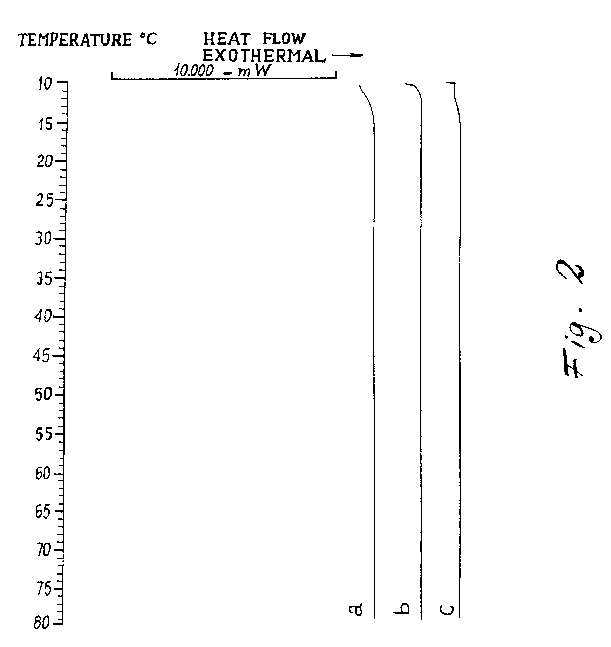

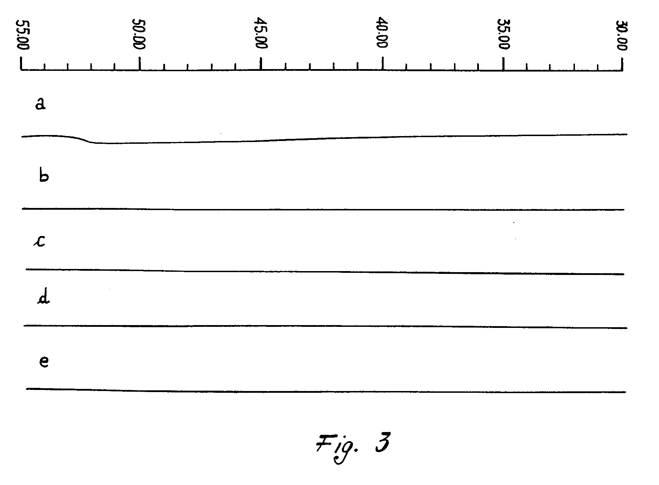

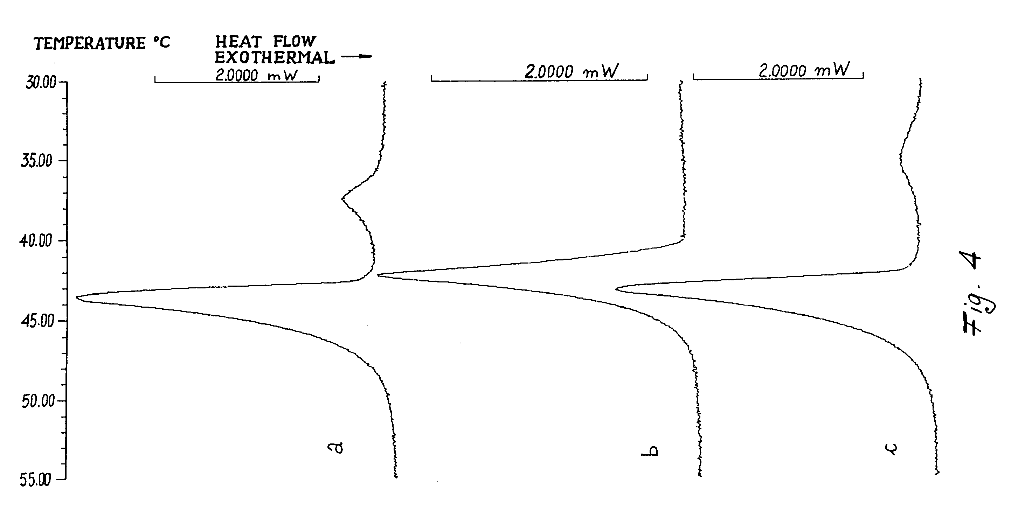

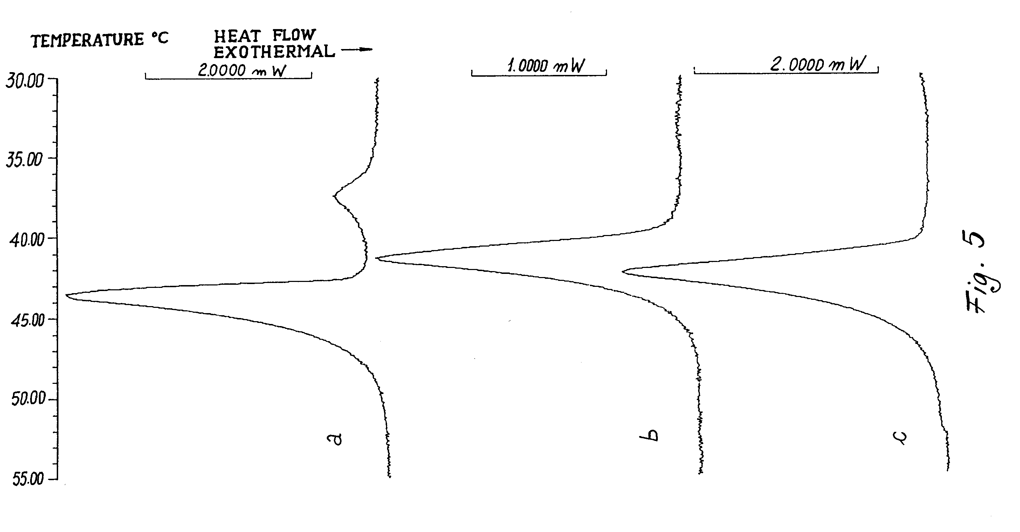

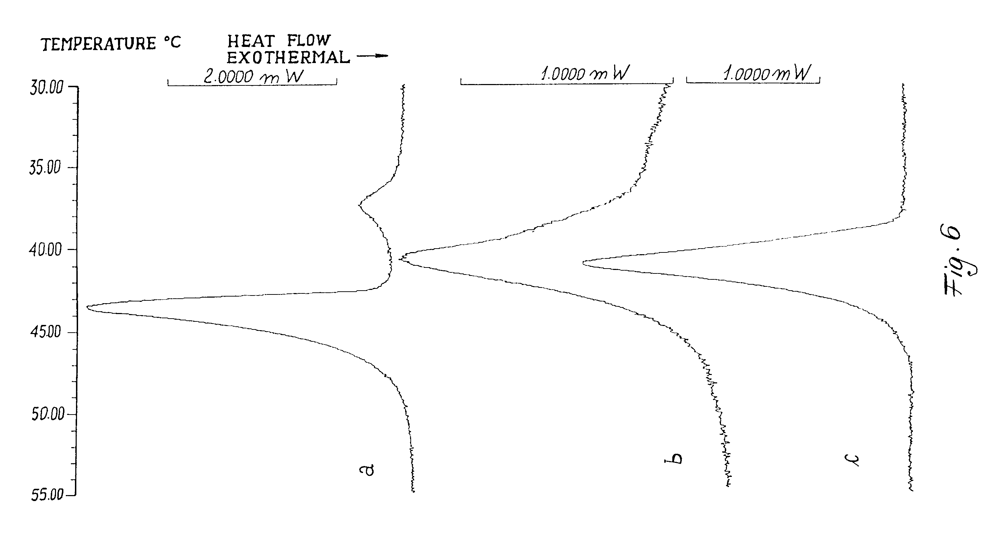

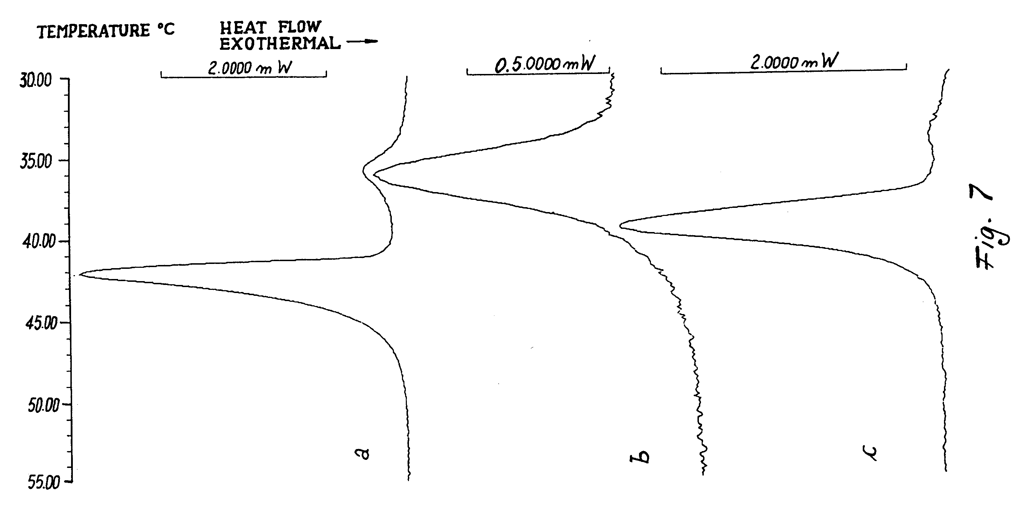

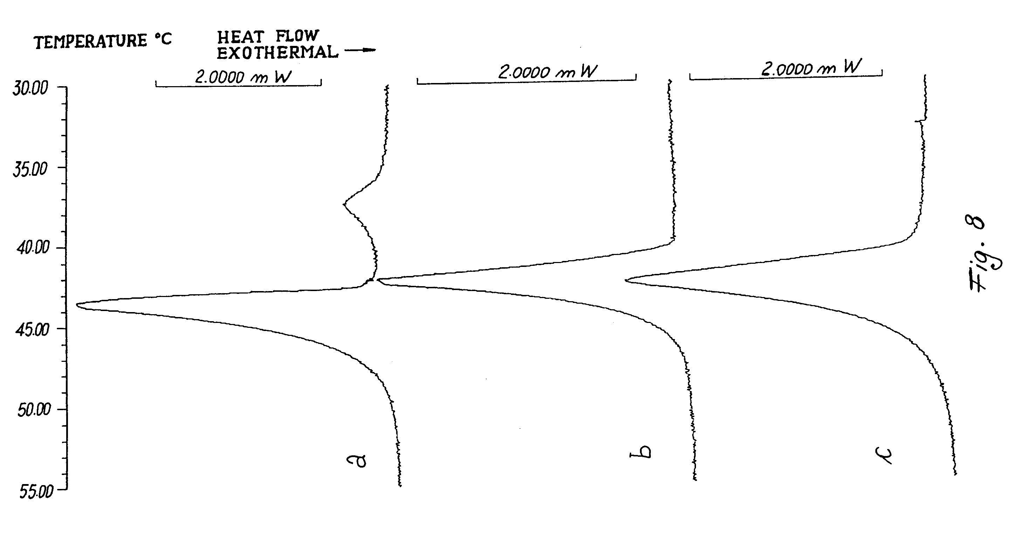

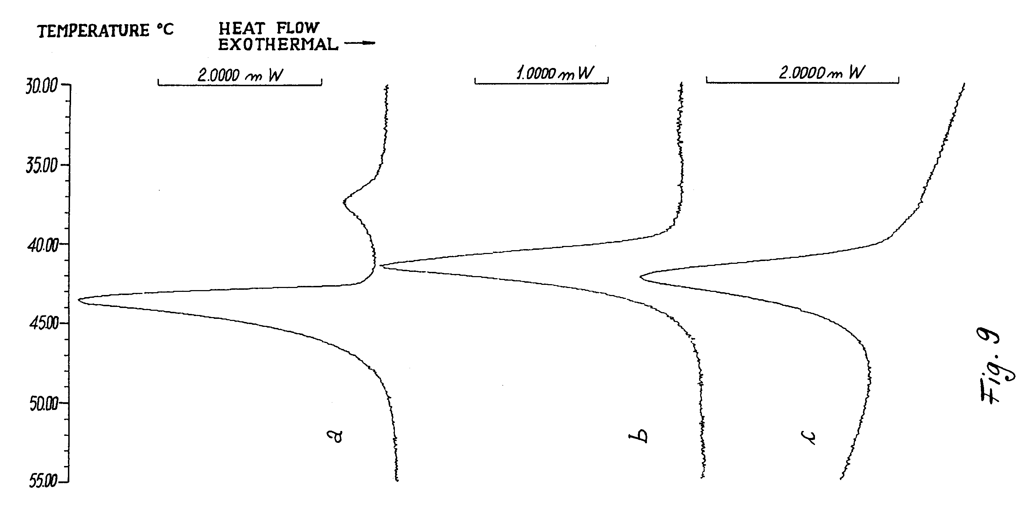

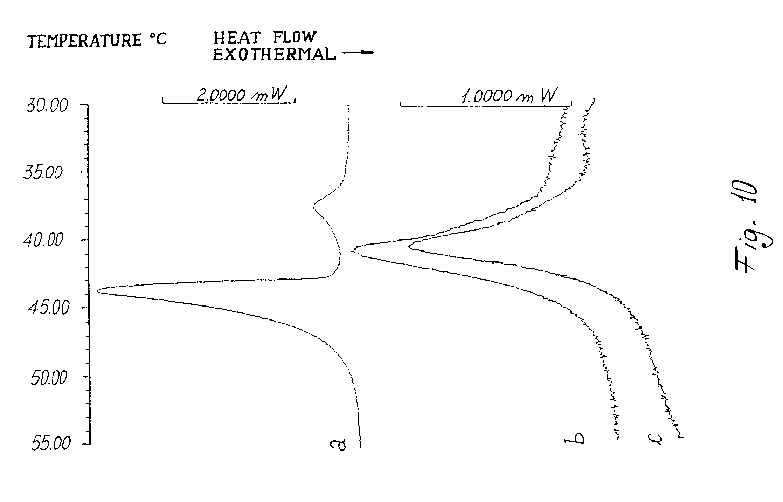

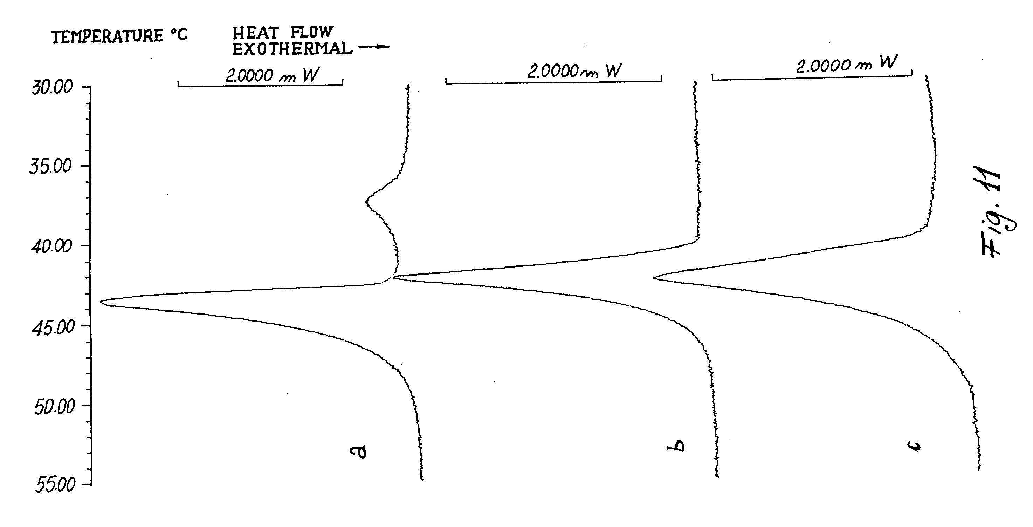

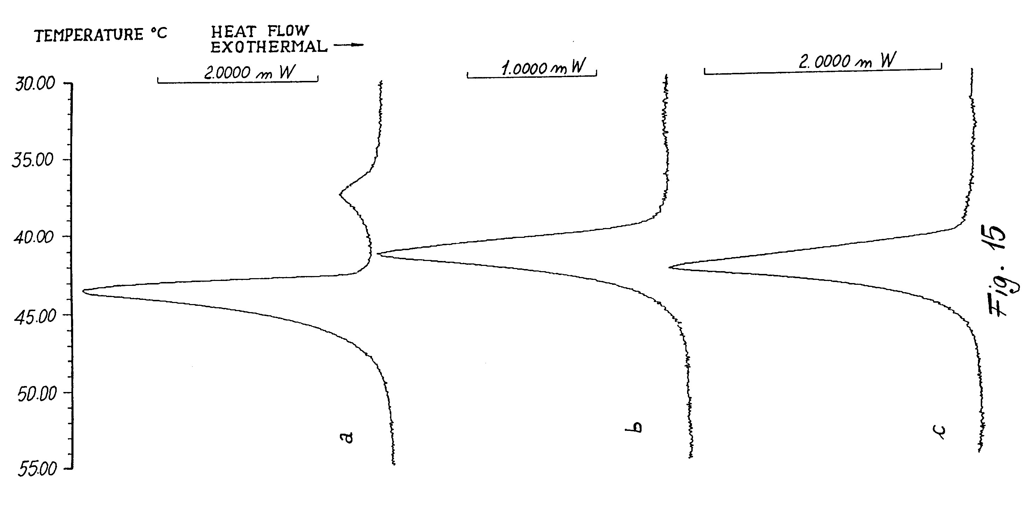

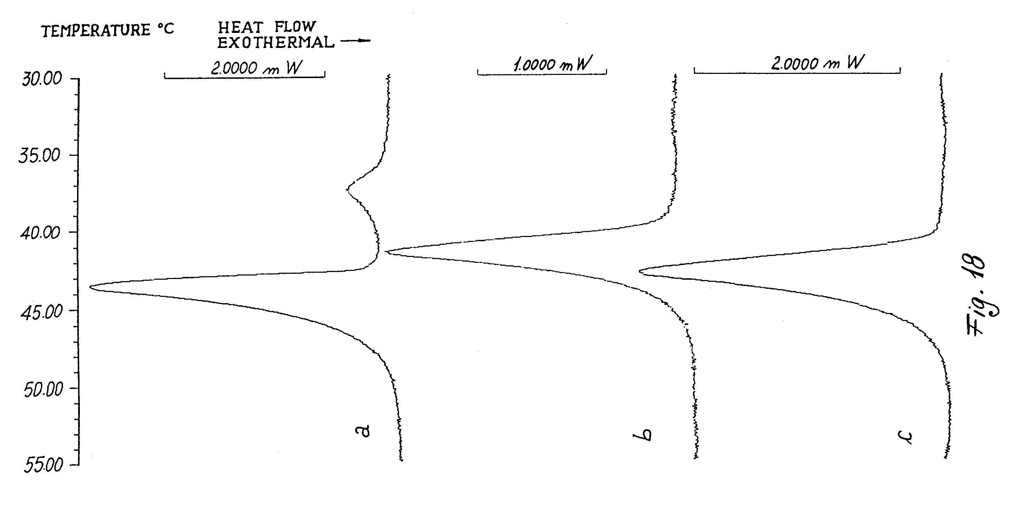

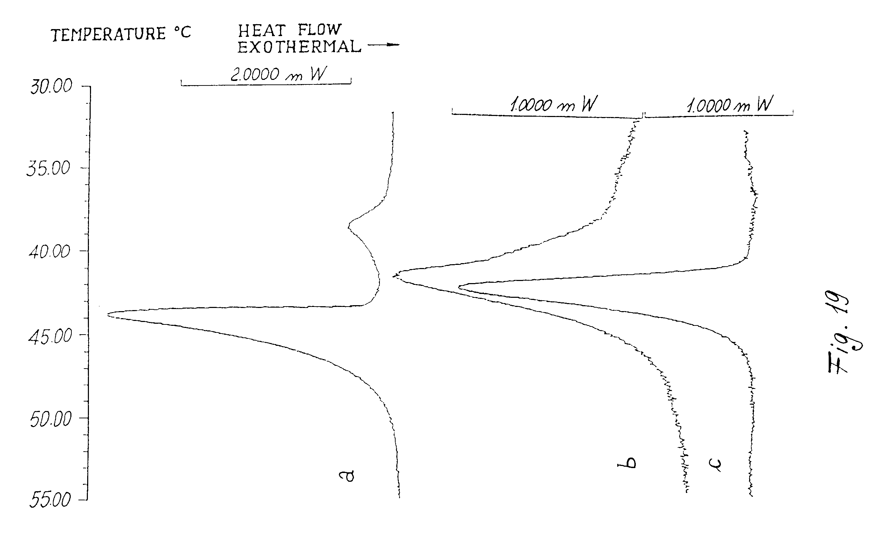

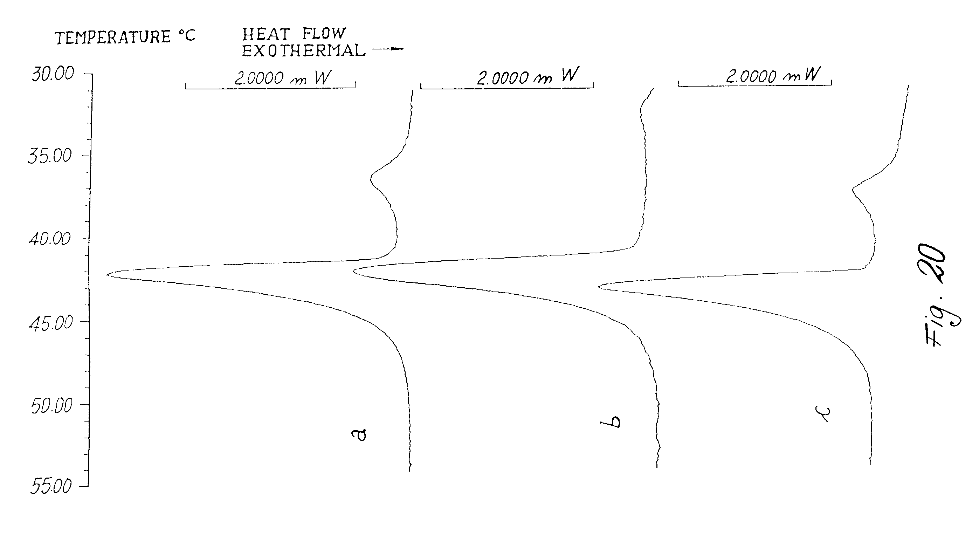

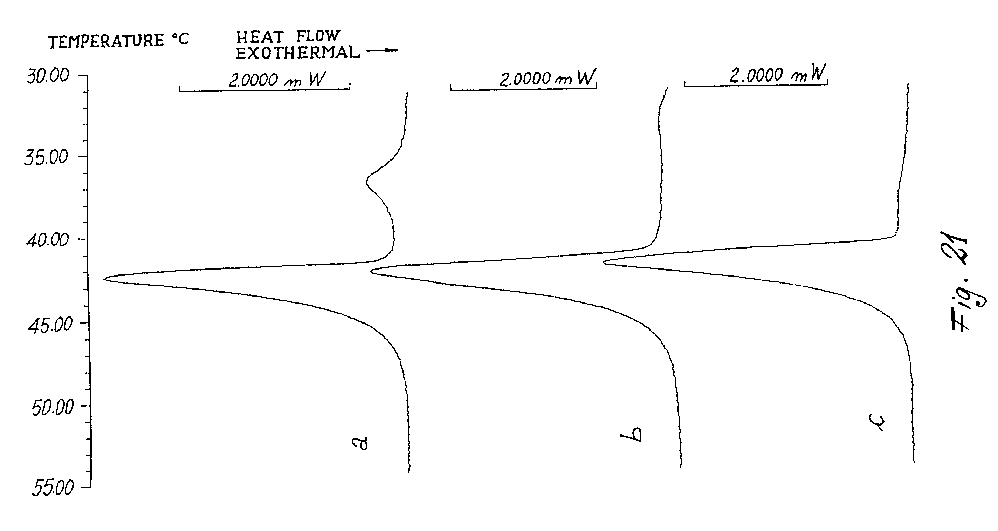

The samples analyzed in DSC were pure substances and their binary and ternary mixtures. The composition of the various samples were as follows :
- Figure 1: Dipalmitoylphosphatidylcholine [DPPC] only;
- Figure 2: a)Laureth 9 [L9]
b)L-α-Lysophosphatidylcholine Palmitoyl [LPC]
c)Deoxycholic Acid Sodium Salt [DCH]
- Figure 3: a)α-cyclodextrin [α-CD]
b) 2-hydroxypropyl-β-cyclodextrin [2-HP/βCD)
c) γ-cyclodextrin [γ-CD]
d) β-cyclodextrin tetradecasulfate [β-CDS]
e) water-soluble polymer of cyclodextrin [CYLCD]
- Figure 4: a)DPPC
b)10/1 mol/mol DPPC/L9
c) 10/1/1 mol/mol/mol DPPC/L9/2HP-βCD
- Figure 5: a)DPPC
b)5/1 mol/mol DPPC/L9
c)5/1/1 mol/mol/mol DPPC/L9/2HP-βCD
- Figure 6: a)DPPC
b)2/1 mol/mol DPPC/L9
c)2/1/1 mol/mol/mol DPPC/L9/2HP-βCD
- Figure 7: a)DPPC
b)1/1 mol/mol DPPC/L9
c)1/1/4 mol/mol/mol DPPC/L9/2HP-βCD
- Figure 8: a)DPPC
b)10/1 mol/mol DPPC/L9
c)10/1/1 mol/mol/mol DPPC/L9/βCDS
- Figure 9: a)DPPC
b)5/1 mol/mol DPPC/L9
c)5/1/1 mol/mol/mol DPPC/L9/βCDS
- Figure 10: a)DPPC
b)2/1 mol/mol DPPC/L9
c)2/1/1 mol/mol/mol DPPC/L9/βCDS
- Figure 11: a)DPPC
b)10/1 mol/mol DPPC/L9
c)10/1/1 mol/mol/mol DPPC/L9/αCD
- Figure 12: a)DPPC
b)5/1 mol/mol DPPC/L9
c)5/1/1 mol/mol/mol DPPC/L9/αCD
- Figure 13: a)DPPC
b)2/1 mol/mol DPPC/L9
c/2/1/1 mol/mol/mol DPPC/L9/αCD
- Figure 14: a)DPPC
b)10/1 mol/mol DPPC/L9
c)10/1/1 mol/mol/mol DPPC/L9/γCD
- Figure 15: a)DPPC
b)5/1 mol/mol DPPC/L9
c)5/1/1 mol/mol/mol DPPC/L9/γCD
- Figure 16: a)DPPC
b)2/1 mol/mol DPPC/L9
c)2/1/1/ mol/mol/mol DPPC/LPC/γCD
- Figure 17: a)DPPC
b)10/1 mol/mol DPPC/L9
c)10/1/1 mol/mol/mol DPPC)L9/CYLCD
- Figure 18: a)DPPC
b)5/1 mol/mol DPPC/L9
c)5/1/1 mol/mol/mol DPPC/L9/CYLCD
- Figure 19: a)DPPC
b)2/1 mol/mol DPPC/L9
c)2/1/1 mol/mol/mol DPPC/L9/CYLCD
- Figure 20: a)DPPC
b)10/1 mol/mol DPPC/LPC
c) 10/1/1/ mol/mol/mol DPPC/LPC/γCD
- Figure 21: a)DPPC
b)5/1 mol/mol DPPC/LPC
c) 5/1/1 mol/mol/mol DPPC/LPC/γCD
- Figure 22: a)DPPC
b)5/1 mol/mol DPPC/LPC
c)5/1/1 mol/mol/mol DPPC/LPC/αCD
- Figure 23: a)DPPC
b)2/1 mol/mol DPPC/DCH
c)2/1/1 mol/mol/mol DPPC/DCH/2HP-βCD
The typical appearance of the DSC curve of sample 1 is shown in Fig. 1. It is evident that two thermal events are observed, an endothermal one at a lower temperature (about 37.5° C) and another endothermal peak at a higher temperature (about 43.5° C). The latter event can be attributed to the gel-liquid crystal transition. The results (DSC curves) are shown and commented in Fig.1 through Fig. 23.
- Figure 1: This is typical DSC curve of DPPC. In which two thermal events are observed. An endothermal pretransition peak at a lower temperature about 37.5 °C and another one transition peak at higher temperature about 43.6°C. The latter transition peak can be attributed to the gel-liquid crystal transition.

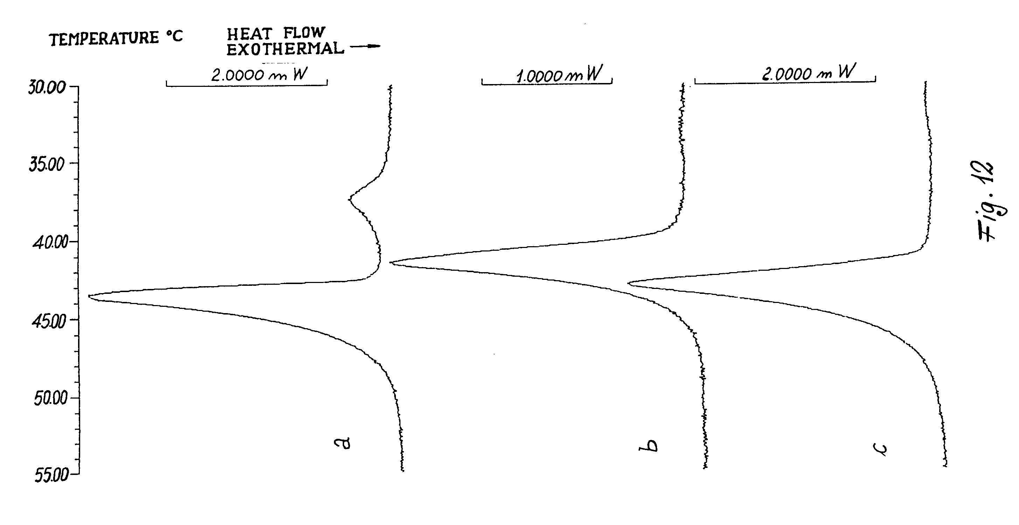

- Figure 2: The enhancers don't give any interference with the DPPC curve.
- Figure 3: The cyclodextrins don't give any interference with DPPC curve. No interference was observed also for binary samples composed of DPPC adding various kinds of cyclodextrins.
- Figure 4: a) DSC curve of DPPC.
b) Addition of Laureth 9 to DPPC gives a lowering of transition peak temperature.
c) Addition of 2-hydroxypropyl-β-cyclodextrin gives a DSC curve of identical pattern as DPPC as is.
- Figure 5: a) DSC curve of DPPC.
b) Addition of Laureth 9 to DPPC gives a lowering of transition peak temperature.
c) Addition of 2-hydroxypropyl-β-cyclodextrin turns the DSC curve more like the pattern of DPPC as is.
- Figure 6: a) DSC curve of DPPC.
b) Addition of Laureth 9 to DPPC gives a lowering and a broadening of transition peak temperature.
c) Addition of 2-hydroxypropyl-β-cyclodextrin turns the DSC curve more like the pattern of DPPC as is.
- Figure 7: a) DSC curve of DPPC.
b) Addition of Laureth 9 to DPPC gives a lowering and a broadening of transition peak temperature.
c) Addition of 2-hydroxypropyl-β-cyclodextrin gives a DSC curve of identical pattern as DPPC as is.
- Figure 8: a) DSC curve of DPPC.
b) Addition of Laureth 9 to DPPC gives a lowering of transition peak temperature.
c) Addition of β-cyclodextrin-tetradecasulfate does not give a DSC curve much more similar to DPPC alone.
- Figure 9: a) DSC curve of DPPC.
b) Addition of Laureth 9 to DPPC gives a lowering of transition peak temperature.
c) Addition of β-cyclodextrin-tetradecasulfate gives origin to modified and complex domains.
- Figure10: a) DSC curve of DPPC.
b) Addition of Laureth 9 to DPPC gives a lowering and a broadening of transition peak temperature.
c) Addition of β-cyclodextrin-tetradecasulfate gives origin to modified and complex domains.
- Figure11: a) DSC curve of DPPC.
b) Addition of Laureth 9 to DPPC gives a lowering of transition peak temperature.
c) Addition of α-cyclodextrin does not give a DSC curve much more similar to DPPC alone.
- Figure12: a) DSC curve of DPPC.
b) Addition of Laureth 9 to DPPC gives a lowering of transition peak temperature.
c) Addition of α-cyclodextrin turns the DSC curve more like the pattern of DPPC as is.

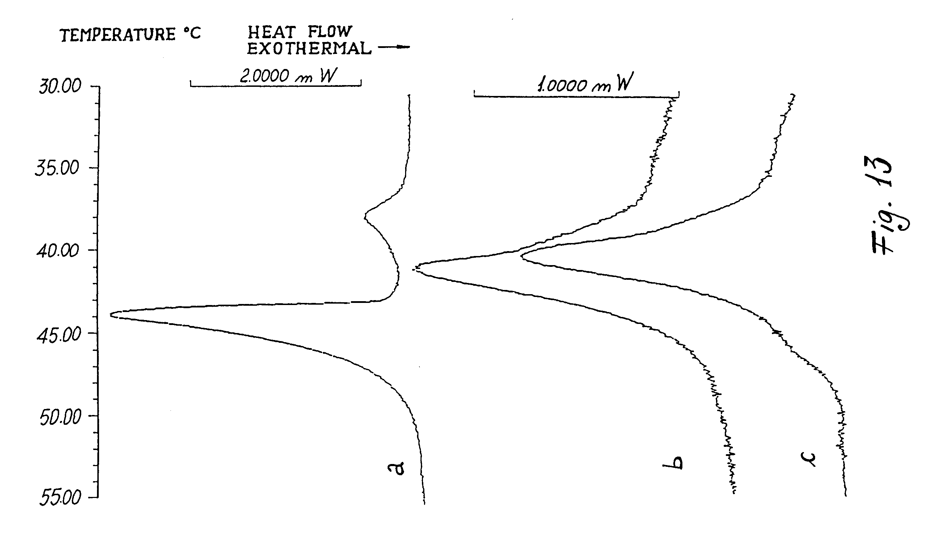

- Figure13: a) DSC curve of DPPC.
b) Addition of Laureth 9 to DPPC gives a lowering and a broadening of transition peak temperature.
c) Addition of α-cyclodextrin gives origin to modified and complex domains.

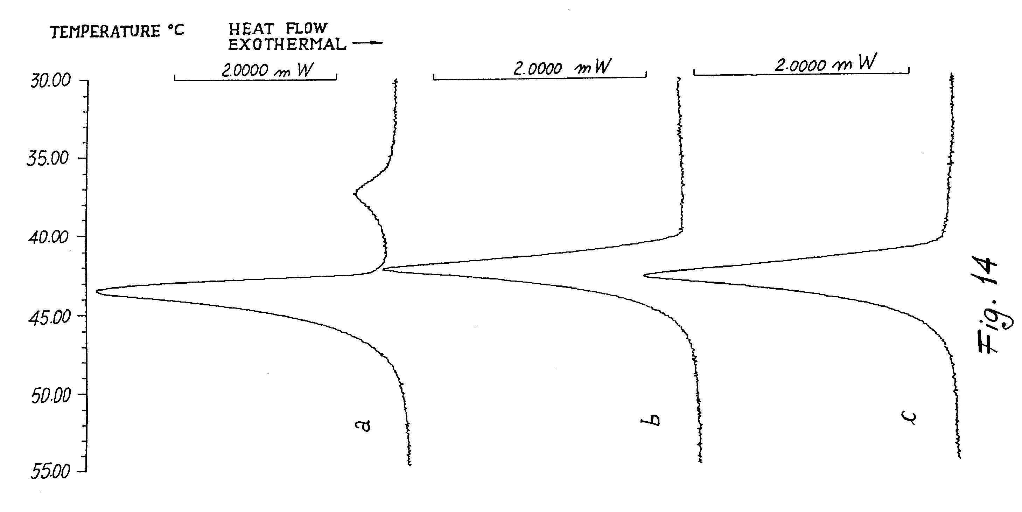

- Figure14: a) DSC curve of DPPC.
b) Addition of Laureth 9 to DPPC gives a lowering of transition peak temperature.
c) Addition of γ-cyclodextrin turns the DSC curve more like the pattern of DPPC as is
- Figure15: a) DSC curve of DPPC.
b) Addition of Laureth 9 to DPPC gives a lowering of transition peak temperature.
c) Addition of γ-cyclodextrin turns the DSC curve more more like the pattern of DPPC as is
- Figure16: a) DSC curve of DPPC.
b) Addition of Laureth 9 to DPPC gives a lowering and a broadening of transition peak temperature.
c) Addition of γ-cyclodextrin turns the DSC curve more more like the pattern of DPPC as is
- Figure17: a) DSC curve of DPPC.
b) Addition of Laureth 9 to DPPC gives a lowering of transition peak temperature.
c) Addition of CYLCD turns the DSC curve more like the pattern of DPPC as is.
- Figure18: a) DSC curve of DPPC.
b) Addition of Laureth 9 to DPPC gives a lowering of transition peak temperature.
c) Addition of CYLCD turns the DSC curve more like the pattern of DPPC as is.
- Figure19: a) DSC curve of DPPC.
b) Addition of Laureth 9 to DPPC gives a lowering and a broadening of transition peak temperature.
c) Addition of CYLCD turns the DSC curve more like the pattern of DPPC as is.

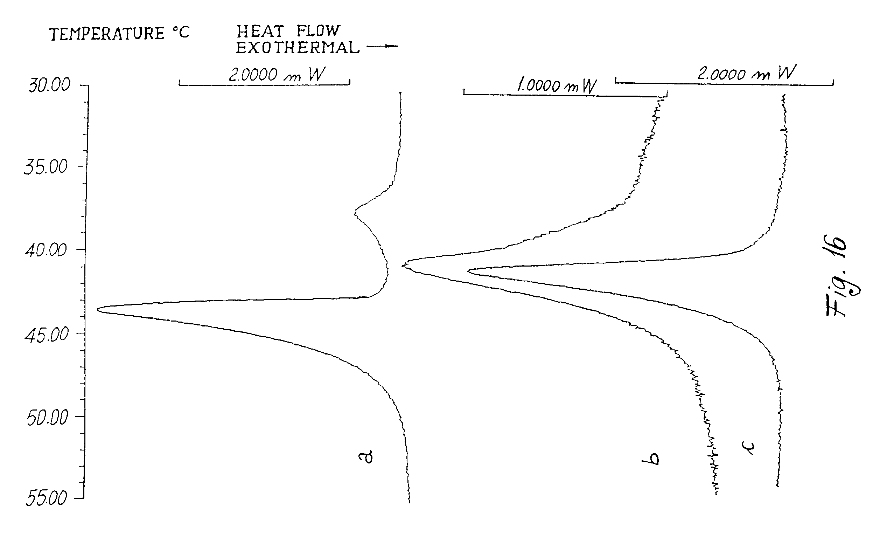

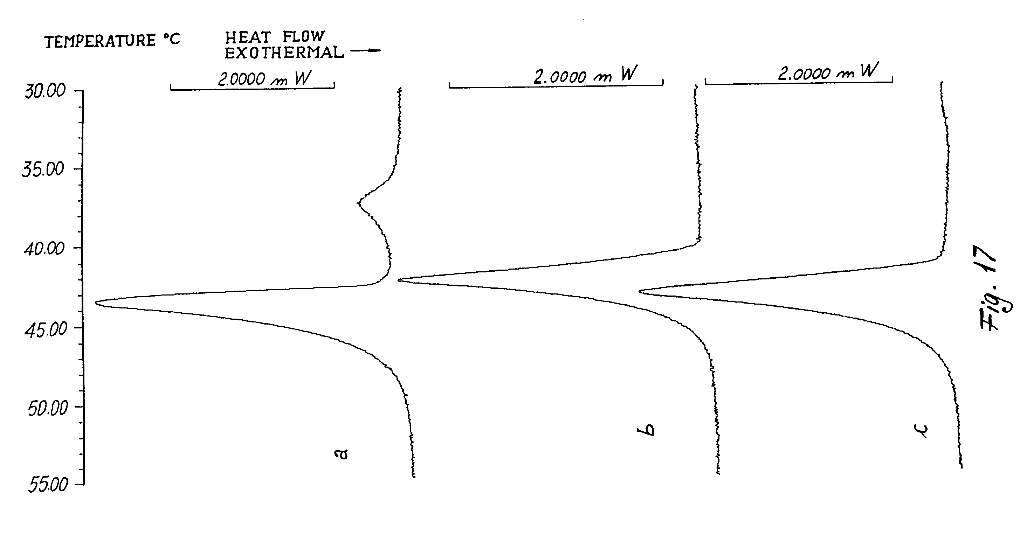

- Figure20: a) DSC curve of DPPC.
b) Addition of LPC to DPPC gives a lowering of transition peak temperature.
c) Addition of γ-cyclodextrin gives a DSC curve of identical pattern of DPPC as is
- Figure21: a) DSC curve of DPPC.
b) Addition of LPC to DPPC gives a lowering of transition peak temperature.
c Addition of γ-cyclodextrin turns the DSC curve more like the pattern of DPPC as is
- Figure22: a) DSC curve of DPPC.
b) Addition of LPC to DPPC gives a lowering and broadening of transition peak temperature.
c) Addition of α-cyclodextrin turns the DSC curve more like the pattern of DPPC as is.

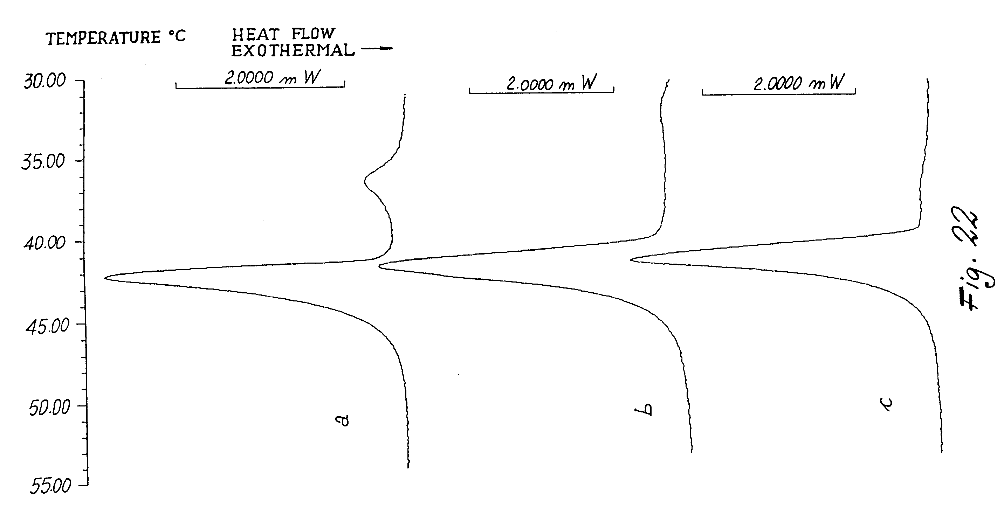

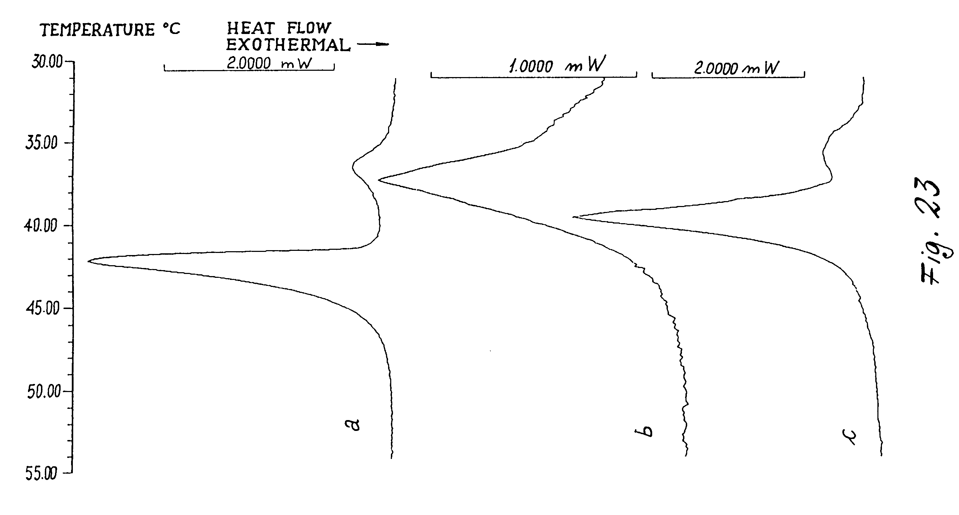

- Figure23: a) DSC curve of DPPC.
b) Addition of DCH to DPPC gives a lowering and a broadening of transition peak temperature.
c) Addition of 2-hydroxyropyl-β-cyclodextrin turns the DSC curve more like the pattern of DPPC as is.

### In vivo experiments

The rat model has been shown to be useful in identifying the histological effects of nasal formulations. A small dose volume administered to one side of the rat nasal cavity only, does not leak into the undosed side. Treated tissue can then be directly compared to untreated tissue in the same animal when complete cross-sections are made.
Groups of four male Wistar rats (Sutton Bonnington, 250g approximately) were used in all experiments. Animals were anaesthetised intraperitoneally with "Sagatal" (sodium pentobarbitone 60 mg/ml; 72 mg/kg) and a tracheotomy performed. 25»l of the nasal formulation was administered to the right nostril of each animal.
The following nasal formulations were administered :
(i) Phosphate Buffer (pH 7.3) .
(ii) Laureth-9 Solution 0.9% w/v in phosphate buffer (9 mg/ml; 0.9 mg/kg in 25 »l).
(iii) 2-Hydroxypropyl-β-Cyclodextrin (2HP-βCD) 81.22 mg/ml in phosphate buffer.
The concentration of HPβC selected was chosen according to the molar ratio of 1:4 (Laureth-9 : 2HP-βCD determined as being the least damaging to red blood cells.
(iv) Laureth-9 0.9% w/v (9 mg/ml) co-administered with 2HP-βCD 81.22 mg/ml in phosphate buffer (mol. ratio 1:4).

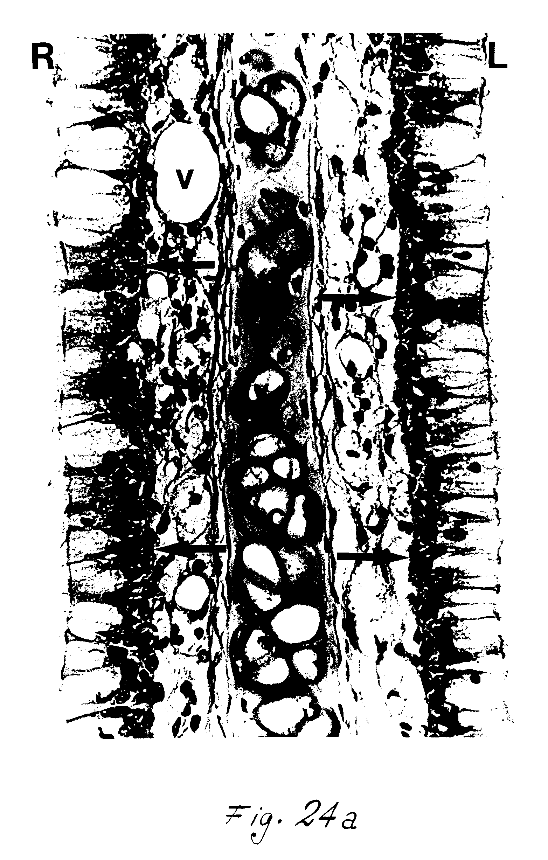

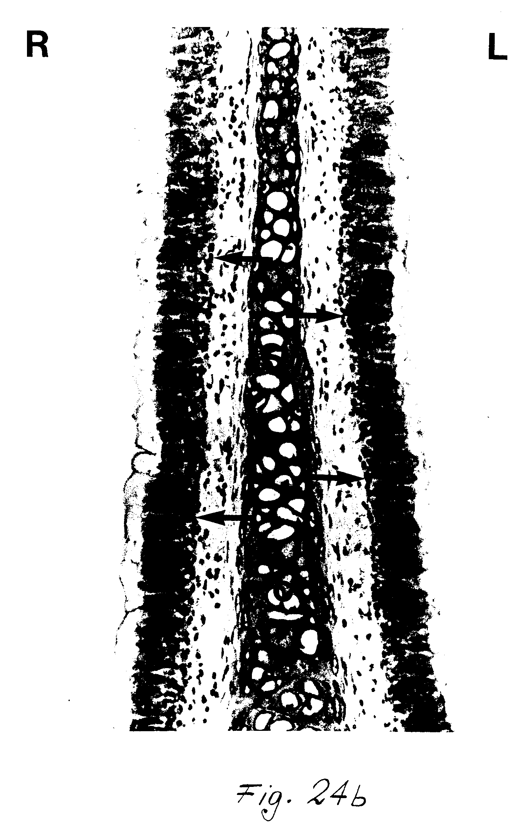

Perfusions was successful in all cases judging by the distribution of yellow fixative.
60 Minutes after administration of the intranasal dose tissue was fixed by cardiac perfusion of a prewash solution (for 2 minutes) followed by Bouin Hollandes aqueous fixative (for 15 minutes). Animals were then decapitated, soft tissue removed and skulls placed in fixative baths overnight.
Specimens were decalcified in 20% EDTA solution for a minimum of 3 weeks after which time region ii of the cavity,between the incisor teeth and incisive papilla, was isolated for further processing. Decalcification was followed by dehydration and wax embedding. Each specimen was orientated so that complete transverse cross-sections of the nasal cavity were produced with the anterior face presented for cutting first. Sections were cut serially at 7»m thickness, mounted and stained with haematoxylin and eosin. Acidic mucopolysaccharides were demonstrated with alcian blue stain (in 3% acetic acid).
Cross-sections were examined with the light microscope. The photomicrographs so obtained are shown in Fig.24 through 27. In each section, tissue on the dosed side of the nasal cavity was compared with that on the undosed control side. In the region investigated, the nasal cavity is lined by a ciliated pseudostratified columnar epithelium on the septum, which is rich in goblet cells (ie respiratory epithelium) while turbinate epithelium is generally thinner, sparsely ciliated and contains few goblet cells.
In all photomicrographs the dosed right side appears on the left and the following key applies :
S = Nasal Septum
Nt = Nasoturbinate
C = Cartilage
G = Goblet Cell
m = mucus
v = vascular sinus
L / R = Left / Right side of the nasal cavity (actual) → = Epithelium-lamina propria junction.
Figure 24a : Photomicrograph of a section through the nasal cavity of a rat dosed with phosphate buffer (pH 7.3) showing ciliated pseudostratified columnar epithelium covering both sides of the nasal septum.
Figure 24b: Photomicrograph of a section through the nasal cavity of a rat dosed with phosphate buffer (pH 7.3) stained with alcian blue to show acidic mucopolysaccharides.

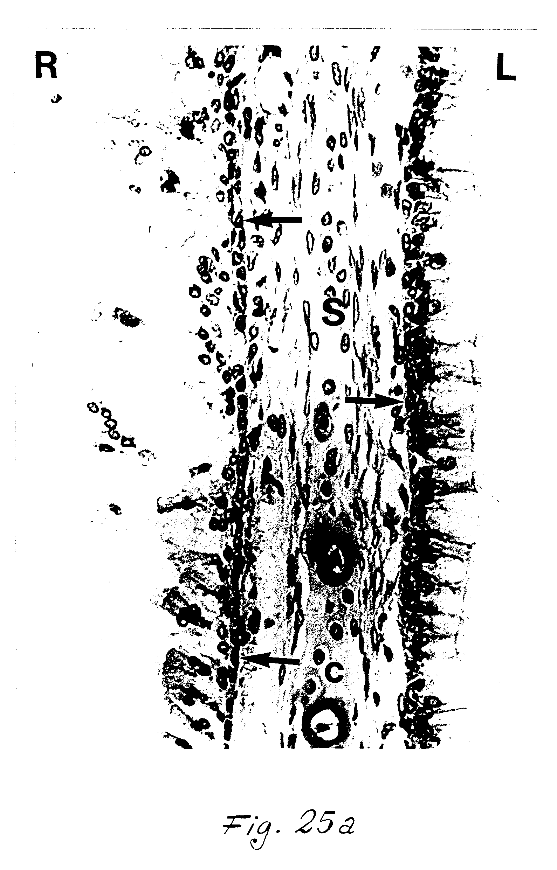

Figure 25a : Photomicrograph of a section through the nasal cavity of a rat doses with Laureth-9 0.9% w/v in phosphate buffer (pH 7.3) showing severe disruption of the epithelium on the dosed side.
Figure 25b : Photomicrograph of a section through the nasal cavity of a rat dosed with Laureth-9 0.9% w/v in phosphate buffer (pH 7.3) stained with alcian blue showing epithelium disruption with loss of mucus from goblet cells on the dosed side.

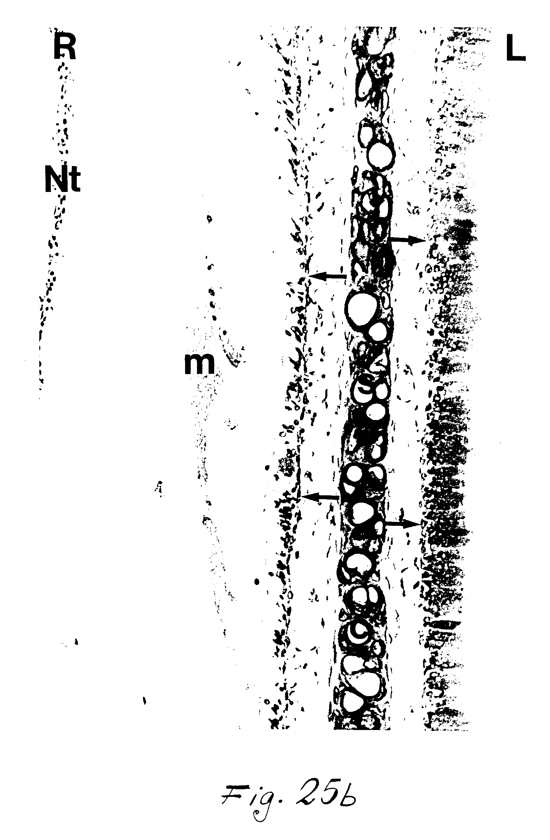

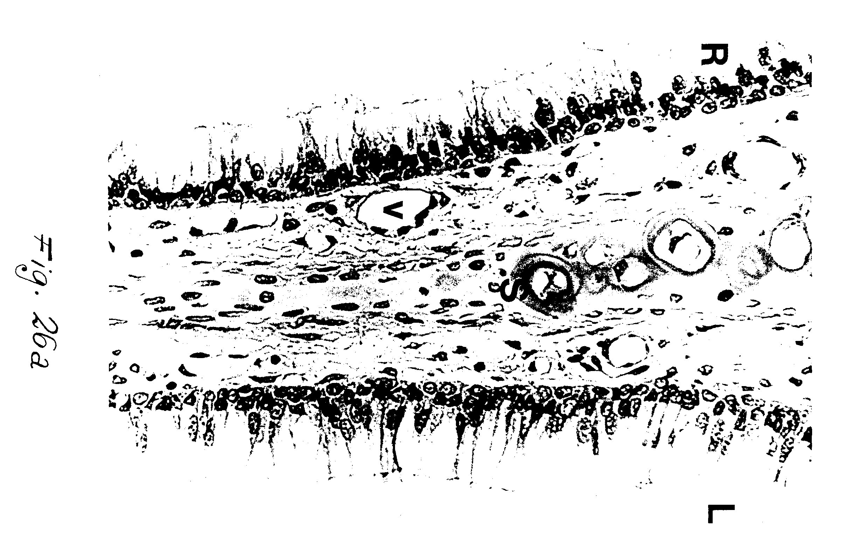

Figure 26a : Photomicrograph of a section through the nasal cavity of a rat dosed with 2HP-βCD 81.22 mg/kg in phosphate buffer (pH 7.3)

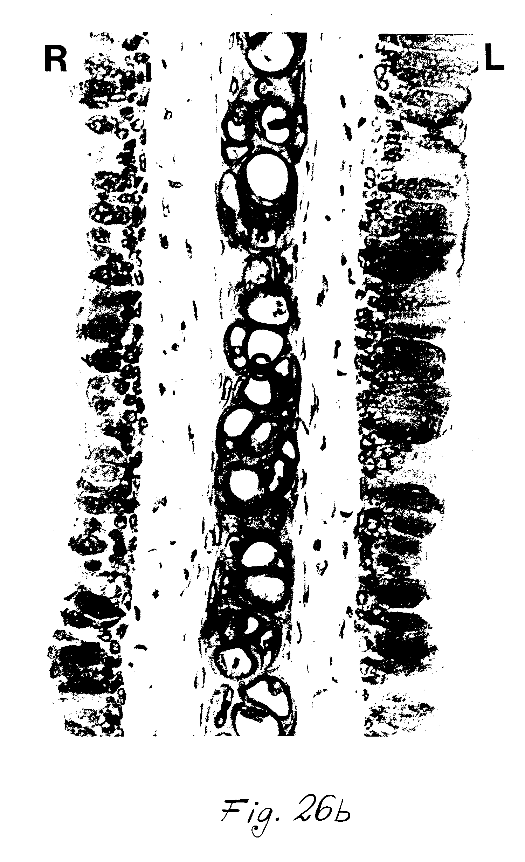

Figure 26b : Photomicrograph of a section through the nasal cavity of a rat doses with 2HP-βCD 81.22 mg/kg in phosphate buffer (pH 7.3) stained with alcian blue showing signs of epithelial interaction on the dosed side.

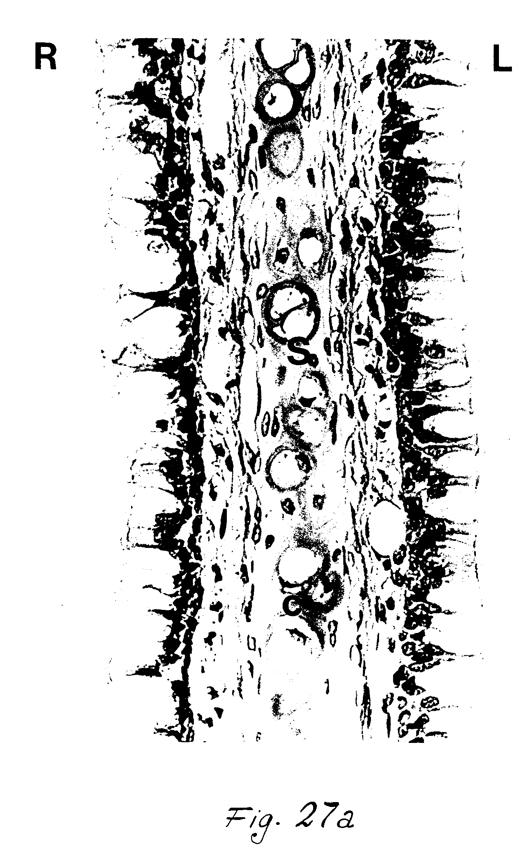

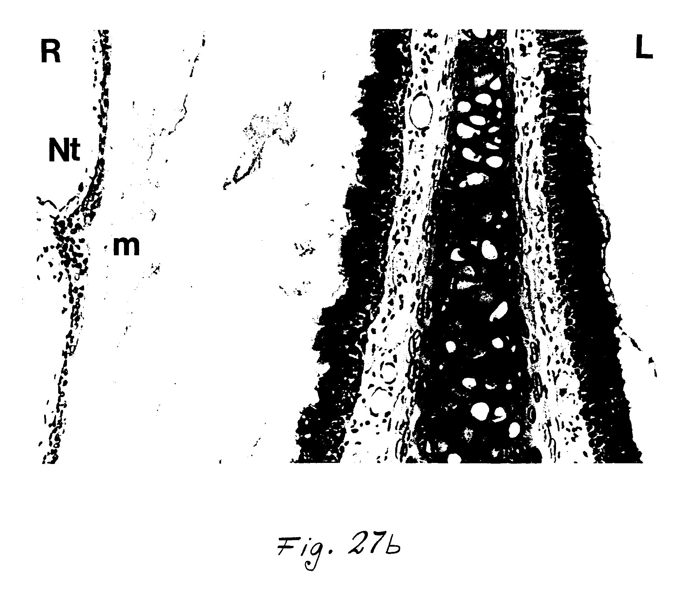

Figure 27a : Photomicrograph of a section through the nasal cavity of a rat dosed with the combined Laureth-9 (0.9% w/v) and 2HP-βCD (81.22 mg/kg) formulation ( in phosphate buffer pH 7.3). Epithelium on the dosed side appears more disordered and cell nuclei are more densely stained and more closely packed towards the basal membrane than on the undosed side.
Figure 27b : Photomicrograph of a section through the nasal cavity of a rat dosed with the combined Laureth-9 (0.9% w/v) and 2HP-βCD (81.22 mg/kg) formulation (in phosphate buffer pH 7.3) stained with alcian blue. Mucus discharged from goblet cells is present in the lumen of the dosed side of the cavity and dosed epithelium is more disordered than that on the undosed side.

The comments to these figures are given herebelow :

### Figure 24

Phosphate buffer resulted in few effects on contact with the nasal epithelium.
There was slightly more mucus present on the luminal surface of the epithelium on the dosed side of the nasal cavity but there was no discernible difference in epithelium thickness on the two side of the septum. The ciliated pseudostratified columnar epithelium appeared the same on both the treated and untreated sides with no obvious signs of interaction with the nasal dose.

### Figure 25

The effects of 0.9% w/v Laureth-9 were severe and widespread in the dosed side of the rat nasal cavity. Treated epithelium was disrupted with significant cell loss and mucus discharge into the lumen of the cavity. This resulted in reduction of the membrane to a thin layer of cells in some places. Effects were observed on the septum, turbinates and lateral wall on the dosed side; epithelium on the undosed side was unaffected.

### Figure 26

Rat nasal tissue exposed to 2HP-βCD showed some signs of interaction between the epithelium and the nasal dose but to a far lesser extent than 0.9% Laureth-9 after one hour exposure. Respiratory tract infection in this group was a particular problem with infected tissue identified in all of the animals, though to varying degrees.
There was a slight reduction in the thickness of the septal epithelium on the dosed side compared to that covering the undosed septum, but no cell loss was observed and cilia were still present on the luminal cell surfaces. Generally there was slightly more mucus discharged into the dosed side of the cavity, except where infection occurred in the undosed side where mucus production was also increased.
On close inspection, the epithelium on the dosed side appeared to be more "disordered" than the control tissue with the line of cilial basal bodies at the luminal surface interrupted at intervals. The nuclei of the epithelial cells on the dosed side tended to be smaller and more irregularly shaped than the large rounded nuclei in the untreated epithelium, particularly over the central septum areas. Also dosed nuclei were stained more densely obscuring intranuclear detail and tended to be more closely packed at the basal membrane. These observations suggest some interaction between dose and tissue, possibly in the early stages.

### Figure 27

The effects of the combined enhancer system were similar to those produced by cyclodextrin alone over 60 minutes, but the disorder of the epithelium and effect on the nuclei was more pronounced, particularly in turbinate epithelium. Again, a complete and relatively thick epithelium was maintained with no surface cell loss, but mucus discharge into the dosed side of the cavity was further increased and epithelium thickness reduced compared with the control tissue.

These observations suggest that the co-administration of 2HP-βCD with Laureth-9 solution in the ratio 4:1 results in a significant decrease in the degree of damage to the nasal epithelium over 60 minutes. Some interaction between the 2HP-βCD and nasal epithelium was indicated by increases in mucus discharge, changes in nuclei appearance and an increase in epithelium "disorder". This latter effect is possibly the result of mucus discharge emptying goblet cells so that their structure becomes more fluid and susceptible to disarrangemet. Such interactions were more marked in the combined formulation bur large scale cell loss such as occurred with Laureth-9 alone in solution, was not observed in any other group of animals.

## Claims

1. A pharmaceutical composition suitable for administering a drug to a mucosal surface of a patient, which composition comprises a drug, an absorption enhancer and a cyclodextrin, excluding
a) compositions wherein the absorption enhancer is at least one of succinic acid, tartaric acid, citric acid fumaric acid, maleic acid, malonic acid, glutaric acid, adipic acid, malic acid, L-glutamic acid, L-aspartic acid, gluconic acid and glucuronic acid and wherein the drug is selected from the group consisting of calcitonin gene related peptides (CGRP), calcitonin, parathyroid hormone (PTH), insulin, somatostatin, growth hormone, secretin, gastrin, vaspressin, oxytocin, glucagon, adrenocorticotropic hormone (ACTH), thyroid-stimulating hormone (TSH), prolactin, luteinizing hormone-releasing hormone (LH-RH), endorphin, enkephalin, neurotensin interferon, interleukin, superoxide dismutase and derivatives and salts thereof;
b) compositions wherein the absorption enhancer is didecanoyl phosphatidylcholine and wherein the drug the drug is selected from the group consisting of insulins, proinsulins, glucagon, parathyroid hormone, parathyroid hormone antagonist, calcitonin, vasopressin, renin prolactin, growth hormone, thyroid stimulating hormone, corticotropin, corticotropin-releasing factor, follicle stimulating hormone, luteinizing hormone, chorionic gonadotropin, atrial peptides, interferon, tissue plasminogen activator, gammaglobulins, Factor VII, Factor VIII, growth hormone releasing hormone, luteinizing hormone releasing hormone, somatostatin and cholecystokinins;
c) composition wherein the drug is 17β-oestradiol, progesterone or a combination thereof and wherein the cyclodextrin is dimethyl-β-ciclodextrin.

2. A composition according to claim 1 in which the absorption enhancer is a nasal absorption enhancer.

3. A composition according to claim 1 or 2 in which the absorption enhancer is present as an inclusion complex of the cyclodextrin.

4. A composition according to any one of the preceding claims in which the cyclodextrin is a soluble cyclodextrin derivative or a swellable polymer of cyclodextrin.

5. A composition according to any one of the preceding claims in which the cyclodextrin is β-cyclodextrin or a β-cyclodextrin derivative.

6. A composition according to claim 5 in which the cyclodextrin is dehydrated β-cyclodextrin or 2-hydroxypropyl β-cyclodextrin.

7. A composition according to any of the preceding claims which further comprises water swellable microspheres or crosslinked starch or destran.

8. A composition according to any one of the preceding claims wherein the drug is a physiologically active polypeptide.

9. A composition according to any one of the preceding claims for use in nasal administration of a drug.

10. Use of a cyclodextrin in the manufacture of a medicament suitable for administering a drug to a mucosal surface of a patient, the said medicament comprising a drug , an absorption enhancer and a cyclodextrin for reducing the toxic effects caused by the absorption enhancer.

11. Use according to claim 10 in the manufacture of a medicament for use in nasal administration of the drug.

12. A process for preparing a composition as claimed in any one of claims 1 to 8 which comprises mixing the drug, the absorption enhancer and the cyclodextrin.

13. A process according to claim 12 which comprises preparing an inclusion complex of the absorption enhancer and the cyclodextrin, and mixing the inclusion complex with the drug.

## Patentansprüche

1. Pharmazeutische Zusammensetzung, die zur Verabreichung eines Arzneimittels an eine Schleimhautoberfläche eines Patienten geeignet ist und die ein Arzneimittel, einen Absorptionsverstärker und ein Cyclodextrin umfaßt, wobei ausgeschlossen sind:
a) Zusammensetzungen, in denen der Absorptionsverstärker mindestens eine Verbindung aus Bernsteinsäure, Weinsäure, Zitronensäure, Fumarsäure, Maleinsäure, Malonsäure, Glutarsäure, Adipinsäure, Äpfelsäure, L-Glutaminsäure, L-Asparaginsäure, Gluconsäure und Glucuronsäure ist und bei denen das Arzneimittel ausgewählt ist aus der Gruppe bestehend aus Calcitonin-Gen-verwandten Peptiden (CGRP), Calcitonin, Parathyroidhormon (PTH), Insulin, Somatostatin, Wachstumshormon, Secretin, Gastrin, Vasopressin, Oxytocin, Glucagon, adrenocorticotropes Hormon (ACTH), Thyroid-stimulierendes Hormon (TSH), Prolactin, luteinisierendes Hormon-freisetzendes Hormon (LH-RH), Endorphin, Enkephalin, Neurotensin, Interferon, Interleukin, Superoxiddismutase und Derivate und Salze davon;
b) Zusammensetzungen, in denen der Absorptionsverstärker Didecanoylphosphatidylcholin ist und das Arzneimittel ausgewählt ist aus der Gruppe bestehend aus Insulinen, Proinsulinen, Glucagon, Parathyroid-Hormonen, Parathyroid-Hormon-Antagonisten, Calcitonin, Vasopressin, Renin, Prolactin, Wachstumshormon, Thyroid-stimulierendem Hormon, Corticotropin, Corticotropin-freisetzendem Faktor, Follikel-stimulierendem Hormon, luteinisierendem-Hormon, Chorion-Gonadotropin, Atrialpeptiden, Interferonen, Gewebeplasminogen-Aktivator, Gammaglobulinen, Faktor VII, Faktor VIII, wachstumshormon-freisetzendem Hormon, luteinisierendes Hormon-freisetzendes Hormon, Somatostatin und Cholecystokininen;
c) Zusammensetzungen, in denen das Arzneimittel 17β-Oestradiol, Progesteron oder eine Kombination davon ist und das Cyclodextrin Dimethyl-β-cyclodextrin ist.

2. Zusammensetzung gemäß Anspruch 1, worin der Absorptionsverstärker ein nasaler Absorptionsverstärker ist.

3. Zusammensetzung gemäß Anspruch 1 oder 2, wobei der Absorptionsverstärker als Einschlußkomplex des Cyclodextrins vorliegt.

4. Zusammensetzung gemäß einem der vorhergehenden Ansprüche, wobei das Cyclodextrin ein lösliches Cyclodextrin-Derivat oder ein quellbares Cyclodextrin-Polymer ist.

5. Zusammensetzung gemäß einem der vorhergehenden Ansprüche, worin das Cyclodextrin β-Cyclodextrin oder ein β-Cyclodextrin-Derivat ist.

6. Zusammensetzung gemäß Anspruch 5, worin das Cyclodextrin dehydratisiertes β-Cyclodextrin oder 2-Hydroxypropyl-β-cyclodextrin ist.

7. Zusammensetzung gemäß einem der vorhergehenden Ansprüche, die außerdem wasserquellbare Mikrokügelchen oder vernetzte Stärke oder Dextran umfaßt.

8. Zusammensetzung gemäß einem der vorhergehenden Ansprüche, worin das Arzneimittel ein physiologisch aktives Polypeptid ist.

9. Zusammensetzung gemäß einem der vorhergehenden Ansprüche zur Verwendung bei der nasalen Verabreichung eines Arzneimittels.

10. Verwendung eines Cyclodextrins bei der Herstellung eines Medikaments, das geeignet zur Verabreichung eines Arzneimittels an eine Schleimhautoberfläche eines Patienten ist, wobei dieses Medikament einen Arzneimittelwirkstoff, einen Absorptionsverstärker und ein Cyclodextrin zur Verringerung der toxischen Wirkungen, die durch den Absorptionsverstärker verursacht werden, umfaßt.

11. Verwendung gemäß Anspruch 10 bei der Herstellung eines Medikaments zur Verwendung bei der nasalen Verabreichung des Arzneimittels.

12. Verfahren zur Herstellung einer Zusammensetzung gemäß einem der Ansprüche 1 bis 8, umfassend das Mischen des Arzneimittels, des Absorptionsverstärkers und des Cyclodextrins.

13. Verfahren gemäß Anspruch 12, umfassend die Herstellung eines Einschlußkomplexes aus Absorptionsverstärker und Cyclodextrin und Mischen des Einschlußkomplexes mit dem Arzneimittel.

## Revendications

1. Composition pharmaceutique convenant pour administrer un médicament sur la surface muqueuse d'un patient, laquelle composition comprend un médicament, un agent favorisant l'absorption et une cyclodextrine, à l'exclusion
a) des compositions dans lesquelles l'agent favorisant l'absorption est au moins un des composés suivants : acide succinique, acide tartrique, acide citrique, acide fumarique, acide maléique, acide malonique, acide glutarique, acide adipique, acide malique, acide L-glutamique, acide L-aspartique, acide gluconique et acide glucuronique, et dans lesquelles le médicament est choisi parmi les peptides se rapportant au gène de la calcitonine (CGRP), la calcitonine, l'hormone parathyroïdienne (PTH), l'insuline, la somatostatine, l'hormone de croissance, la sécrétine, la gastrine, la vaspressine, l'oxytocine, le glucagon, l'hormone adrénocorticotropique (ACTH), l'hormone stimulant la thyroïde (TSH), la prolactine, l'hormone de libération de l'hormone lutéinisante (LH-RH), l'endorphine, l'encéphaline, la neurotensine, l'interféron, l'interleukine, la superoxyde dismutase, et leurs dérivés et leurs sels ;
b) des compositions dans lesquelles l'agent favorisant l'absorption est la didécanoyl phosphatidylcholine et dans lesquelles le médicament est choisi parmi les insulines, les proinsulines , le glucagon, l'hormone parathyroïdienne, l'antagoniste de l'hormone parathyroïdienne, la calcitonine, la vasopressine, la rénine, la prolactine, l'hormone de croissance, l'hormone stimulant la thyroïde, la corticotropine, le facteur de libération de la corticotropine, l'hormone stimulant le follicule, l'hormone lutéinisante, la gonadotrophine corionique, les peptides auriculaires, l'interféron, l'activateur du plasminogène des tissus, les gamma-globulines, le facteur VII, le facteur VIII, l'hormone libérant l'hormone de croissance, l'hormone libérant l'hormone lutéinisante, la somatostopine et les cholécystokinines ;
c) de la composition dans laquelle le médicament est le 17β-oestradiol, la progestérone ou une association de ceux-ci et dans laquelle la cyclodextrine est la diméthyl-β-cyclodextrine.

2. Composition selon la revendication 1, dans laquelle l'agent favorisant l'absorption est un agent favorisant l'absorption nasale.

3. Composition selon la revendication 1 ou 2, dans laquelle l'agent favorisant l'absorption est présent sous la forme d'un complexe d'inclusion de la cyclodextrine.

4. Composition selon l'une quelconque des revendications précédentes, dans laquelle la cyclodextrine est un dérivé soluble de la cyclodextrine ou un polymère gonflable de la cyclodextrine.

5. Composition selon l'une quelconque des revendications précédentes, dans laquelle la cyclodextrine est la β-cyclodextrine ou un dérivé de la β-cyclodextrine.

6. Composition selon la revendication 5, dans laquelle la cyclodextrine est la β-cyclodextrine déshydratée ou la 2-hydroxypropyl β-cyclodextrine.

7. Composition selon l'une quelconque des revendications précédentes, qui comprend en outre des microsphères gonflables dans l'eau, ou de l'amidon réticulé ou du dextranne réticulé.

8. Composition selon l'une quelconque des revendications précédentes, dans laquelle le médicament est un polypeptide physiologiquement actif.

9. Composition selon l'une quelconque des revendications précédentes destinée à l'utilisation pour l'administration nasale d'un médicament.

10. Utilisation d'une cyclodextrine dans la fabrication d'un produit médicamenteux convenant pour administrer un médicament sur la surface muqueuse d'un patient, ce produit médicamenteux comprenant un médicament, un agent favorisant l'absorption et une cyclodextrine pour réduire les effets toxiques causés par l'agent favorisant l'absorption.

11. Utilisation selon la revendication 10 pour la fabrication d'un produit médicamenteux pour l'utilisation pour l'administration, nasale du médicament.

12. Procédé de préparation d'une composition selon l'une quelconque des revendications 1 à 8, qui comprend le mélange du médicament, de l'agent favorisant l'absorption et de la cyclodextrine.

13. Procédé selon la revendication 12, qui comprend la préparation d'un complexe d'inclusion de l'agent favorisant l'absorption et de la cyclodextrine, et le mélange du complexe d'inclusion avec le médicament.
